# EUROPEAN PATENT APPLICATION

(11) **EP 1 103 246 A1**
(43) Date of publication of application: **30.05.2001**
(21) Application number: 00124727.9
(22) Date of filing: 13.11.2000
(51) Int. Cl.: A61K 7/035

(54) **Anhydrous powder compositions**

(30) Priority: 15.11.1999 US 165633 P
(71) Applicant: Cognis Corporation, Gulph Mills, PA 19406 (US)
(72) Inventor: Day, Rupert P., Connecticut, US 06418 (US)
(74) Representative: Fabry, Bernd

(57) **Abstract**

The present invention recommends a powder composition comprising
(a) a solid particulate component and
(b) chitosan having a particle size of from 0.1 to 50 microns.

## Description

### Preamble

The invention refers to anhydrous powder compositions comprising chitosan, a process for treating human skin and the use of said powders for making decorative cosmetic compositions.

### Background of the invention

The present invention generally relates to powder cosmetic compositions having enhanced tactile and wear properties. More particularly, the present invention relates to the preparation of loose and/or compacted powder formulations containing chitosan which increase the bulk density of particulate powders in a manner which increases the surface area covered by the powder when it is applied onto a substrate and, when used in powder cosmetic compositions, imparts improved feel properties onto human skin.

It is known that some cosmetic compositions such as blushes, eye shadows, face powders or foundations are provided in the form of compacted or cast powders. These are anhydrous compositions commonly referred to as "compact powders". They mainly consist of a mixture of colored or non-colored powders and a fatty binder, such as an oil or a mixture of oil and wax, which are shaped by compression or by casting into a container serving as a mould. These powders are generally used by removing a small quantity of powder and then applying it to the skin by means of an applicator such as a sponge, powder puff or brush.

The preparation of binding agents in such compact powders poses various problems. The final product should be sufficiently homogeneous and compact in order to avoid the fragmentation caused especially by impact, while retaining good disintegration capacity. Moreover, the composition should have a smooth feel and should be easy to spread in a continuous manner. In addition, the binder should be compatible with pigments and specialists are familiar with the problems of degradation of certain pigments when conventional fatty binders are used.

Certain make-up compositions are also provided in the form of "loose powders", wherein the particles are neither compacted nor dispersed in a fatty continuous phase, but retain on the contrary their individuality. Such loose powders often contain a fatty substance (oil) whose role is especially to increase the smoothness of application to promote the adherence of the powder to the skin, and to allow solubilisation of some active ingredients. Some loose powders may contain relatively high quantities of oil without the particles having a tendency to agglomerate. Such is the case especially for powders containing particles in the form of hollow microspheres made from synthetic thermoplastic materials. However, the formulation of such loose powders poses the above-mentioned problem relating to the degradation of certain pigments in the presence of the fatty substances conventionally used.

### Description of the invention

The present invention is directed to an anhydrous powder composition containing:
(a) a solid particulate component; and
(b) a chitosan having a particle size of from 0.01 to 50 microns.

The present invention is also directed to a process for treating skin involving contacting the skin with a powder composition containing:
(a) a solid particulate component; and
(b) a chitosan having a particle size of from 0.1 to 50 microns.

There are a number of suitable solid particulate components which may be employed in the present invention including, but not limited to, talc, micas, modified or unmodified starch, silica, alumina, boron nitride, kaolin, zinc and titanium oxides, stearates, precipitated calcium carbonates, magnesium carbonate or hydrocarbonate, metallic soaps derived from a carboxylic organic acid having from 8 to 22 carbon atoms, synthetic polymer or (copolymer) powders such as polyethylenes, polyacrylates, polymethacrylates, polyesters, polyamides, and the like, and powders in the form of hollow microspheres made from thermoplastic materials whose hollow part may contain a gas.

### Chitosan

Chitosan is derived from chitin by deacetylation. Chitin itself is typically obtained from conventional sources such as crustacean outer shells and fungal mycelial mats. Chitin and chitosan refer to a family of compounds that exhibit widely differing physical and chemical properties. These differences are due primarily to the products' varying molecular weights, degrees of acetylation and presence of contaminants such as covalently bound, species-specific proteins, single amino acid and inorganic contaminants. Though any type of chitosans, in general, may be used by the present invention, particularly preferred chitosans are those having molecular weights ranging from 1,000 to 25,000,000, and preferably from 500,000 to 5,000,000 g/mol, and a particle size of from 0.1 to 50 microns, preferably from 0.1 to 40, and most preferably from 0.1 to 10 microns.

### Anhydrous powder compositions

According to one embodiment of the present invention, there is provided an anhydrous powder composition containing:
(a) from 10 to 95% by weight, preferably from 50 to 95% by weight, and most preferably from 70 to 95% by weight of a solid particulate component; and
(b) from 0.01 to 10% by weight, preferably from 0.1 to 5% by weight, and most preferably from 0.1 to 3% by weight of a chitosan, all weights being based on the weight of the composition.

The anhydrous powder composition described above can either be used by itself as, for example, a cosmetic foundation for human skin, or it may be combined with other additives for various other applications.

For instance, in order to impart color onto a substrate, such as human skin, treated therewith, pigments can be added to the base composition in an amount of from 0.01 to 60% by weight, preferably from 2 to 20% by weight, and most preferably from 5 to 20% by weight, based on the weight of the composition.

### Pigments

Suitable pigments may be chosen from inorganic and/or organic pigments, and/or pearlescent pigments. Examples of inorganic pigments include, but are not limited to, titanium dioxide, black, yellow, red and brown iron oxides, manganese violet, ultramarine violet, ultramarine blue, chromium oxide, hydrated chromium oxide and ferric blue. Organic pigments which may be employed include, but are not limited to, D & C red No. 3, D & C red No. 6, D & C red No. 7, D & C red No. 9, D & C red No. 13, D & C red No. 19, D & C red No. 21, D & C red No. 27, D & C red No. 30, D & C red No. 36, carbon black and lacquers based on carmine. Suitable pearlescent pigments include, but are not limited to, white pearlescents such as mica coated with titanium oxide or bismuth oxychloride, mica-titanium colored with iron oxides, mica-titanium colored with ferric blue, and the like.

### Binder and emollients

Another auxiliary component which may be included, particularly in the event that a compacted rather than loose cosmetic composition is being formed is a binder/emollient which acts to both further enhance compaction/binding of the solid particulate component and provide emolliency properties onto skin treated therewith. Suitable binder/emollients which may be employed by the present invention include, but are not limited to, fatty acids, fatty alcohols, esters of fatty acids and fatty alcohols, Guerbet alcohols, waxes, silicones, alkanes, vegetable oils, mineral oils, animal oils such as lanolin, insect oils, humectants, glycols, starches, sugars, excipients, plasticizers, aromatic hydrocarbons, deionized water, aliphatic hydrocarbons, cyclic hydrocarbons, and the like. In the event that a binder/emollient is employed, it can be present in the composition in an amount of from 0.05 to 50% by weight, preferably from 1 to 10% by weight, and most preferably from 3 to 8% by weight, based on the weight of the composition.

### Additives

Various standard additives may also be incorporated into the invention including, but not limited to, preservatives; antiseptics such as trichlorodiphenyl ethers, boric acid, and the like) which are used especially in deodorant powders for the body and feet and in baby powders; astringent agents which are used in deodorant powders and in foot powders such as aluminum hydroxychloride and alum; anti-perspirants; sunscreen agents; cicatrizing agents; anti-free radical agents; vitamins, demulcent agents; perfumes; consistency agents; anti-pruritics (anti-itch) and the like. In the event that an additive is employed in the composition of the present invention, it will typically be present in an amount of from 0.01 to 50% by weight, based on the weight of the composition.

### Process for treating skin

According to another embodiment of invention, there is provided a process for treating skin involving contacting the skin with the powder composition of the present invention. The precise amount of powder composition to be applied will depend on the end use of the product and can be easily determined by those skilled in the art.

The powder compositions of the present invention can be prepared in any conventional manner. For example, for compacted powders all of the components may be mixed and then compacted using a press. For cast powders, the components can be mixed and suspended in a solvent. This mixture is then cast into a form and the solvent evaporated therefrom. For loose powders, the components can be mixed together and then ground up.

### Use of the anhydrous powders

The anhydrous powder composition of the present invention contains, preferably, two basic components, namely, a solid particulate component and, a chitosan. This powder composition may then be used as a cosmetic composition, preferably a decorative cosmetic composition, such as, for example, make-up, eyeshadow and lipstick, a foot powder, a deodorant, and the like.

The present invention will be better understood from the examples which follow, all of which are intended for illustrative purposes only and are not meant to unduly limit the scope of the invention in any way.

### Examples

### Example 1.

A loose powder composition was prepared containing the following components:

| | |
|---|---|
| talc | 93.95 % b.w. |
| zinc stearate | 5.00 % b.w. |
| chitosan | 0.50 % b.w. |
| preservative | 0.55 % b.w. |

The talc and zinc stearate were added to an blender and blended for 2 minutes on high speed (grind). The preservative was then added and blended, at the same speed, for an additional 2 minutes. The chitosan was then added and also blended, at the same speed, for 2 minutes. It was observed that the chitosan appeared to be effectively dispersed throughout the loose powder composition and appeared to also impart a nice feel property onto human skin. Some degree of adhesion of the powder also appeared to exist.

### Example 2.

A powder composition was prepared containing the following components:

| | |
|---|---|
| talc | 70.05 % b.w. |
| zinc stearate | 6.50 % b.w. |
| chitosan | 0.50 % b.w. |
| preservative | 0.55 % b.w. |
| oil binder | 6.50 % b.w. |
| color pigments | 0.90 % b.w. |
| pearl pigment | 15.00 % b.w. |

The talc and zinc stearate and pigments were added to an blender and blended for 4-5 minutes on high speed (grind). The preservative was then added and blended, at the same speed, for an additional 2 minutes. The chitosan was then added and blended, at the same speed, for 2 minutes. Half of the oil binder was then slowly added and blended for 3 minutes. The other half of the oil binder was then and blended for an additional 3 minutes. The side walls of the blender were then scraped and the mixture was then blended for an additional 4 minutes until uniform. It was observed that the chitosan appeared to be effectively dispersed throughout the anhydrous powder composition and appeared to also impart an exceptional feel property onto human skin. An excellent degree of adhesion was also observed.

### Example 3.

A powder composition was prepared containing the following components:

| | |
|---|---|
| talc | 69.55 % b.w. |
| zinc stearate | 6.50 % b.w. |
| chitosan | 1.00 % b.w. |
| preservative | 0.55 % b.w. |
| oil binder | 6.50 % b.w. |
| color pigments | 0.90 % b.w. |
| pearl pigment | 15.00 % b.w. |

The talc and zinc stearate and color pigments were added to an blender and blended for 4-5 minutes on high speed (grind). The preservative was then added and blended, at the same speed, for an additional 2 minutes. The chitosan was then added and blended, at the same speed, for 2 minutes. Half of the oil binder was then slowly added and blended for 3 minutes. The other half of the oil binder was then and blended for an additional 3 minutes. The side walls of the blender were then scraped and the mixture was then blended for an additional 4 minutes until uniform. It was observed that the chitosan appeared to be effectively dispersed throughout the anhydrous powder composition and appeared to also impart an exceptional feel property onto human skin. An excellent degree of adhesion also observed.

### Examples 4 and 5.

Table 1 refers to a liquid make-up/foundation (light coverage) of o/w type, which shows a very light, almost transparent coverage. Table 2 reflects a hair care dry color formulae.

Manufacturing/Processing/Mixing/Procedure: PHASE A : Keltrol and Veegum were added to water, and then homogenized for 10 to 15 minutes. The remaining phase A ingredients were then blended in. The mixture was then heated to 75 °C . PHASE B : All ingredients comprising PHASE B were then mixed together and heated to 75°C. Phase A and Phase B were then combined. PHASE C : Phase A and Phase B were then blended in an Osterizer until uniform color was achieved. PHASE D : The active ingredients were then added at cooling on slow sweep. Slowly add Phase D. Cool and package at a pour temperature. Viscosity may be increased or reduced by increasing or decreasing the emulsifiers or thickeners.

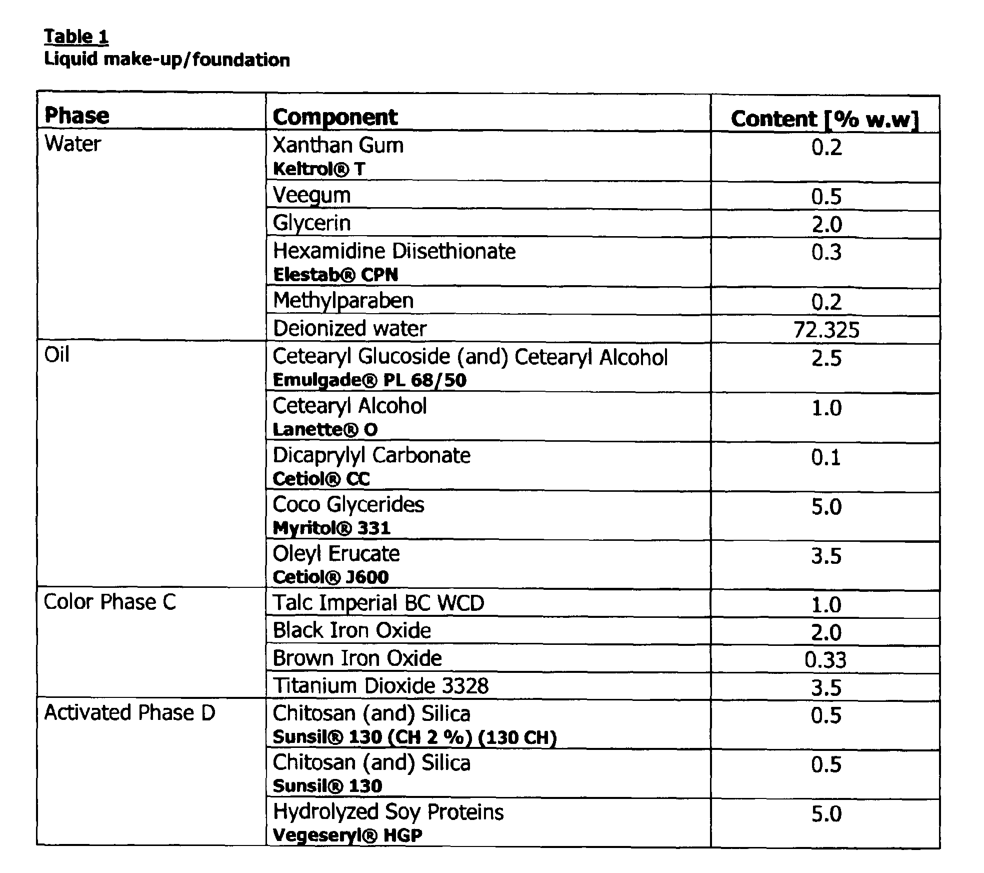

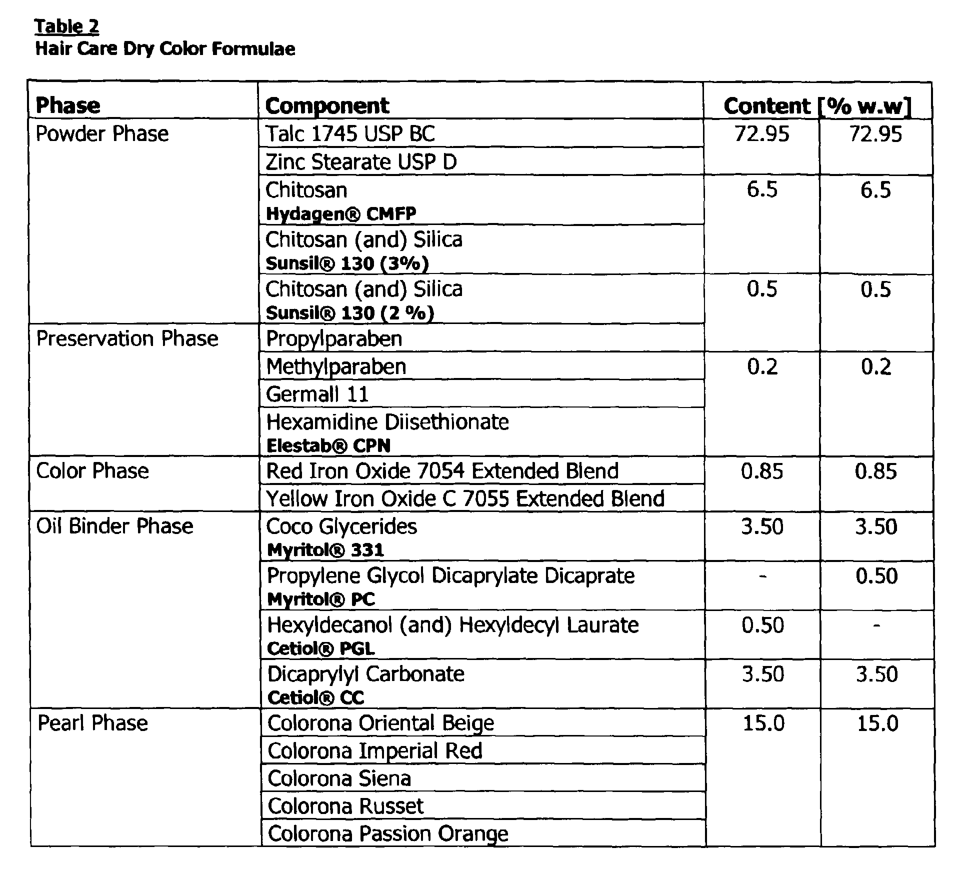

## Claims

1. A powder composition comprising
(a) a solid particulate component and
(b) chitosan having a particle size of from 0.1 to 50 microns.

2. The composition according to claim 1, **characterized in** that the solid particulate component is present in the composition in an amount of from 10 to 95% by weight, based on the total weight of the composition.

3. The composition according to claims 1 and/or 2, **characterized in** that further pigments are present.

4. The composition according to any of the claims 1 to 3, **characterized in** that further binder components are present.

5. The composition according to any of the claims 1 to 4, **characterized in** that further additives are present selected from the group consisting of preservatives, antiseptics, astringent agents, sunscreen agents, cicatrizing agents, anti-free radical agents, vitamins, demulcent agents, perfumes, consistency agents, anti-pruritics, anti-perspirants, and mixtures thereof.

6. A powder composition comprising:
(a) from 70 to 95% by weight of a solid particulate;
(b) from 0.01 to 3% by weight of a chitosan having a particle size of from 1 to 10 microns;
(c) from 10 to 20% by weight of a pigment; and
(d) from 3 to 8% by weight of a binder,
all weights being based on the total weight of the composition.

7. The composition according to claim 6, **characterized in** that further additives are present selected from the group consisting of preservatives, antiseptics, astringent agents, sunscreen agents, cicatrizing agents, anti-free radical agents, vitamins, demulcent agents, perfumes, consistency agents, and mixtures thereof.

8. A process for treating skin comprising contacting the skin with a powder composition containing a solid particulate component and chitosan having a particle size of less than 400 microns.

9. The process according to claim 8, **characterized in** that the solid particulate component is present in the composition in an amount of from 10 to 95% by weight, based on the total weight of the composition.

10. The use of a powder composition comprising
(a) a solid particulate component and
(b) chitosan having a particle size of from 0.1 to 50 microns
for making decorative cosmetic products.
